# NEUE EUROPÄISCHE PATENTSCHRIFT

(11) **EP 0 931 054 B2**
(45) Veröffentlichungstag und Bekanntmachung des Hinweises auf die Entscheidung über den Einspruch: **11.07.2012**
(45) Hinweis auf die Patenterteilung: 28.05.2003
(21) Anmeldenummer: 97942889.3
(22) Anmeldetag: 28.08.1997
(51) Int. Cl.: C07C 209/86

(54) **VERFAHREN ZUR ABTRENNUNG VON 2-AMINOMETHYLCYCLOPENTYLAMIN AUS EINER MISCHUNG ENTHALTEND HEXAMETHYLENDIAMIN UND 2-AMINOMETHYLCYCLOPENTYLAMIN**
PROCESS FOR THE SEPARATION OF 2-AMINOMETHYLCYLOPENTYLAMINE FROM A MIXTURE CONTAINING HEXAMETHYLDIAMINE AND 2-AMINOMETHYLCYCLOPENTYLAMINE
PROCEDE POUR LA SEPARATION DE 2-AMINOMETHYLCYCLOPENTYLAMINE DANS UN MELANGE CONTENANT DE L'HEXAMETHYLENEDIAMINE ET DE LA 2-AMINOMETHYLCYCLOPENTYLAMINE

(30) Priorität: 10.09.1996 DE 19636764; 07.02.1997 DE 19704617
(43) Veröffentlichungstag der Anmeldung: 28.07.1999
(73) Patentinhaber: BASF SE, 67056 Ludwigshafen (DE)
(72) Erfinder: LUYKEN, Hermann, D-67069 Ludwigshafen (DE); REHFINGER, Alwin, D-67112 Mutterstadt (DE); BASSLER, Peter, D-68519 Viernheim (DE); VOIT, Guido, D-69198 Schriesheim (DE); FISCHER, Rolf, D-69121 Heidelberg (DE); MERGER, Martin, D-67227 Frankenthal (DE); RUST, Harald, D-67435 Neustadt (DE)
(74) Vertreter: Féaux de Lacroix, Stefan
(86) Internationale Anmeldenummer: PCT/EP1997/004687
(87) Internationale Veröffentlichungsnummer: WO 1998/011052

(56) Entgegenhaltungen:
- EP-A- 0 319 434
- GB-A- 731 819
- US-A- 3 696 153
- DATABASE WPI Section Ch, Week 198149, Derwent Publications Ltd., London, GB; Class A41, AN 1981-90805D, XP002050218 & SU 810 669 A (KULIK L N) 09 April 1981
- 'Report no. 31, Process Economics Program', 1967, STANFORD RESEARCH INSTITUTE, MENLO PARK, CALIFORNIA, US Artikel PARK L. MORSE: 'Hexamethylene Diamine', Seiten 111 - 128A
- 'Report no. 54B, Process Economics Program', September 1987, SRI INTERNATIONAL, MENLO PARK, CALIFORNIA, US Artikel YEN-CHEN, YEN, ET AL: 'Nylon 6 6', Seiten 149 - FF

## Beschreibung

Die vorliegende Erfindung betrifft ein Verfahren zur Abtrennung von 2-Aminomethylcyclopentylamin (AMCPA) aus einer Mischung enthaltend Hexamethylendiamin und AMCPA.

Die vollständige Hydrierung von Adipodinitril zu Hexamethylendiamin, sowie die partielle Hydrierung unter gleichzeitiger Herstellung von Hexamethylendiamin und 6-Amincapronitril, in Gegenwart eines Katalysators auf der Basis eines Metalls wie Nickel, Cobalt, Eisen, Rhodium oder Ruthenium ist allgemein bekannt, beispielsweise aus: K. Weissermel, H.-J. Arpe, Industrielle Organische Chemie, 3. Auflage, VCH Verlagsgesellschaft mbH, Weinheim, 1988, Seite 266, US-A 4 601 859, US-A 2 762 835, US-A 2 208 598, DE-A 848 654, DE-A 954 416, DE-A 42 35 466, US-A 3 696 153, DE-A 19500222, WO 92/21650 und der Deutschen Anmeldung 19548289.1.

Als Nebenprodukte entstehen unter anderem Hexamethylenimin (HMI), 1,2-Diaminocyclohexan (DACH) und AMCPA.

Aus US-A 3 696 153 ist bekannt, daß das durch diese Hydrierung erhaltene Hexamethylendiamin als Nebenprodukt AMCPA enthält, das sich extrem schwer von der Mischung abtrennen läßt.

Aus DE-A 1 543 973 ist bekannt, das die Reinigung von rohem, durch diese Hydrierung erhaltenem Hexamethylendiamin durch Destillation nicht sehr wirkungsvoll ist.

Aufgabe der vorliegenden Erfindung war es daher, ein Verfahren bereitzustellen, das die Abtrennung von AMCPA von einer Mischung, die im wesentlichen Hexamethylendiamin und AMCPA enthält, auf technisch einfache und wirtschaftliche Weise ermöglicht.

Demgemäß wurde ein Verfahren zur Abtrennung von AMCPA aus einer Mischung enthaltend im wesentlichen Hexamethylendiamin und AMCPA, dadurch gekennzeichnet, daß man die Trennung destillativ bei einem Druck von 1 bis 200 mbar durchführt, wobei der Druck im Sumpf der Destillationsapparatur zwischen 3 und 200 mbar und der Druck im Kopf der Destillationsapparatur zwischen 1 und 200 mbar beträgt gefunden.

Die partielle oder vollständige Hydrierung von Adipodinitril kann man nach einem der bekannten Verfahren durchführen, beispielsweise nach einem der zuvor genannten Verfahren beschrieben in US 4 601 859, US 2 762 835, US 2 208 598, DE-A 848 654, DE-A 954 416, DE-A 4 235 466, US-A 3 696 153, DE-A 9 500 222 oder WO 92/21650, indem man im allgemeinen die Hydrierung in Gegenwart von Nickel-, Cobalt-, Eisen- oder Ruthenium-haltigen Katalysatoren durchführt. Dabei können die Katalysatoren als. Katalysatoren durchführt. Dabei können die Katalysatoren als. Trägerkatalysatoren oder als Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht.

Bei der Hydrierung erhält man eine Mischung, die Hexamethylendiamin, AMCPA und gegebenenfalls 6-Aminocapronitril und Adipodinitril enthält.

Die Abtrennung von einer Mischung enthaltend Hexamethylendiamin und AMCPA kann in an sich bekannter Weise, vorzugsweise destillativ, beispielsweise gemäß der DE-A 195 002 22 oder der Deutschen Anmeldung 19 548 289.1, gleichzeitig oder nacheinander erfolgen, beispielsweise in einer Destillationskolonne oder in einer Seitenabzugskolonne mit Trennwand (Petlyuk-Kolonne), so daß die Kondensationstemperatur der nachfolgenden Reinigungskolonne bei der AMCPA-Trennung in der Regel höher und die Kondensation erleichtert ist. Die leichtsiedende Nebenkomponente DACH kann, sofern vorhanden, vorteilhaft gleichzeitig mit AMCPA abgetrennt werden, die Abtrennung von DACH kann aber auch in einer separaten Kolonne über Kopf erfolgen.

Vorteilhaft trennt man vor der Reinigung des Hexamethylendiamins nach dem erfindungsgemäßen Verfahren leichtsiedende Nebenkomponenten, wie HMI, sofern vorhanden, vollständig oder teilweise ab.

In die Mischung enthaltend Hexamethylendiamin und AMCPA beträgt vor der Trennung im allgemeinen der Gewichtsanteil an AMCPA, bezogen auf Hexamethylendiamin, 10 bis 10000 ppm, insbesondere 100 bis 1000 ppm, wobei das erfindungsgemäße Verfahren nicht auf diese Grenzen beschränkt ist.

Die Abtrennung von AMCPA aus der Mischung führt man erfindungsgemäß destillativ bei Drücken von zwischen 1 und 200 mbar durch. Erfindungsgemäß beträgt der Druck im Sumpf der Destillationsapparatur zwischen 3 und 200 mbar und der Druck im Kopf der Destillationsapparatur zwischen 1 und 200 mbar. Wird die Abtrennung von AMCPA aus des Mischung destillativ bei Drücken von zwischen 1 und 300 mbar durch geführt, dass ergeben sich Sumpftemperaturen im Bereich von 40 bis 160°C.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen oder Füllkörperkolonnen.

Bevorzugt sind Destillationsapparturen, die vom Sumpf zum Kopf einen Druckabfall von 0 bis 200 mbar, vorzugsweise 0 bis 50 mbar aufweisen, wobei der Druck im Sumpf erfindungsgemäß im Bereich von 3 bis 200 mbar, und im Kopf im Bereich von 1 bis 200 mbar, liegt.

Besonders geeignet sind Destillationskolonnen, die einen geringen Druckverlust, vorzugsweise höchstens 1 mbar, insbesondere 0,5 mbar pro theoretischer Trennstufe aufweisen.

Insbesondere geeignet sind Packungskolonnen, vorzugsweise mit geordneten Füllkörpern wie Metallblechpackungen, insbesondere Drahtgewebepackungen.

Nach dem erfindungsgemäßen Verfahren wird das AMCPA als Kopfprodukt erhalten, im allgemeinen, sofern im Gemisch enthaltend im wesentlichen Hexamethylendiamin und AMCPA vorhanden, im Gemisch mit DACH und leichtsiedenden Komponenten.

Die Destillation kann man in mehreren, wie 2 oder 3 Kolonnen, vorteilhaft einer einzigen Kolonne durchführen.

Hexamethylendiamin kann mit Dicarbonsäuren wie Adipinsäure zu technisch bedeutenden Polymeren verarbeitet werden.

### Beispiele

### Beispiel 1

10 kg/h HMD mit einem AMCPA-Gehalt von 300 ppm wurden einer Kolonne mit druckverlustarmer Gewebepackung entsprechend 100 theoretischen Trennstufen zugeführt. Am Kopf der Kolonne wurde ein Druck von 100 mbar eingestellt, im Sumpf wurde ein Druck von 140 mbar gemessen. Am Kopf der Kolonne wurden konstant 6 g/h Produkt abgenommen und 50 kg/h als Rücklauf der Kolonne zurückgeführt.

Der Gehalt an AMCPA des als Sumpfprodukt erhaltenen Hexamethylendiamins lag unter der Nachweisgrenze von 1 ppm, der Gehalt an Hexamethylendiamin des Kopfproduktes betrug 50,2 Gew.-%.

### Vergleichsbeispiel 1

Es wurde wie in Beispiel 1 Verfahren mit der Ausnahme, daß am Kopf der Kolonne ein Druck von 250 mbar eingestellt und am Sumpf ein Druck von 305 mbar gemessen wurde.

Der Gehalt an AMCPA des als Sumpfprodukt erhaltenen Hexamethylendiamins betrug 11 ppm, der Gehalt an Hexamethylendiamin des Kopfproduktes betrug 51,9 Gew.-%.

### Vergleichsbeispiel 2

Es wurde wie in Beispiel 1 Verfahren mit der Ausnahme, daß am Kopf der Kolonne ein Druck von 500 mbar eingestellt und am Sumpf ein Druck von 545 mbar gemessen wurde.

Der Gehalt an AMCPA des als Sumpfprodukt erhaltenen Hexamethylendiamins betrug 48 ppm, der Gehalt an Hexamethylendiamin des Kopfproduktes betrug 58,2 Gew.-%.

### Vergleichsbeispiel 3

10 kg/h HMD mit einem AMCPA-Gehalt von 300 ppm wurden einer Siebbodenkolonne mit 140 Siebböden zugeführt. Am Kopf der Kolonne wurde ein Druck von 100 mbar eingestellt, im Sumpf wurde ein Druck von 900 mbar gemessen. Am Kopf der Kolonne wurden konstant 6 g/h Produkt abgenommen und 50 kg/h als Rücklauf der Kolonne zurückgeführt.

Der Gehalt an AMCPA des als Sumpfprodukt erhaltenen Hexamethylendiamins betrug 22 ppm, der Gehalt an Hexamethylendiamin des Kopfproduktes betrug 53,7 Gew.-%

## Patentansprüche

1. Verfahren zur Abtrennung von 2-Aminomethylcyclopentylamin aus einer Mischung enthaltend Hexamethylendiamin und 2-Aminomethylcyclopentylamin, **dadurch gekennzeichnet, daß** man die Trennung destillativ bei einem Druck von 1 bis 200 mbar durchführt wobei der Druck im Sumpf der Destillationsapparatur zwischen 3 und 200 mbar, und der Druck im Kopf der Destillationsapparatur zwischen 1 und 200 mbar beträgt.

2. Verfahren nach Anspruch 1, wobei man als Destillationsapparatur eine Destillationskolonne einsetzt.

3. Verfahren nach Anspruch 1 oder 2, wobei man als Destillationsapparatur eine Packungskolonne einsetzt.

4. Verfahren nach den Ansprüchen 1 bis 3, wobei man als Destillationsapparatur eine Destillationskolonne einsetzt, die einen Druckverlust von höchstens 1 mbar pro theoretischer Trennstufe aufweist.

5. Verfahren nach den Ansprüchen 1 bis 4, wobei der Gewichtsanteil an 2-Aminomethylcyclopentylamin, bezogen auf Hexamethylendiamin, vor der Trennung 1 bis 10000 ppm beträgt.

6. Verfahren nach den Ansprüchen 1 bis 5, wobei der Gewichtsanteil an 2-Aminomethylcyclopentylamin, bezogen auf Hexamethylendiamin, nach der Trennung 0 bis 100 ppm beträgt.

## Claims

1. A process for separating 2-aminomethylcyclopentylamine from a mixture comprising hexamethylenediamine and 2-aminomethylcyclopentylamine, which comprises distilling at from 1 to 200 mbar, wherein the pressure at the bottom of the distillation apparatus is within the range from 3 to 200 mbar and the pressure at the top of the distillation apparatus is within the range from 1 to 200 mbar.

2. A process as claimed in claim 1, wherein the distillation apparatus used is a distillation column.

3. A process as claimed in any of claims 1 or 2, wherein the distillation apparatus used is a packed column.

4. A process as claimed in any of claims 1 to 3, wherein the distillation apparatus used is a distillation column with a pressure drop of not more than 1 mbar per theoretical plate.

5. A process as claimed in any of claims 1 to 4, wherein the weight proportion of 2-aminomethylcyclopentylamine, based on hexamethylenediamine, is within the range from 1 to 10,000 ppm prior to the separation.

6. A process as claimed in any of claims 1 to 5, wherein the weight proportion of 2-aminomethylcyclopentylamine, based on hexamethylenediamine, is within the range from 0 to 100 ppm after the separation.

## Revendications

1. Procédé de séparation de 2-aminométhylcyclopentylamine à partir d'un mélange contenant de l'hexaméthylènediamine et de la 2-aminométhylcyclopentylamine, **caractérisé en ce qu'**on effectue la séparation par distillation à une pression de 1 à 200 mbars, dans lequel la pression dans le fond de l'appareil de distillation s'élève entre 3 et 200 mbars et la pression dans la tête de l'appareil de distillation s'élève entre 1 et 200 mbars.

2. Procédé suivant revendication 1, dans lequel on met en oeuvre, comme appareil de distillation, une colonne de distillation.

3. Procédé suivant l'une des revendications 1 et 2, dans lequel on met en oeuvre, comme appareil de distillation, une colonne à garnissage.

4. Procédé suivant l'une des revendications 1 à 3, dans lequel on met en oeuvre, comme appareil de distillation, une colonne de distillation qui présente une perte de pression d'au maximum 1 mbar par étage de séparation théorique.

5. Procédé suivant l'une des revendications 1 à 4, dans lequel la fraction pondérale de 2-aminométhylcyclopentylamine par rapport à l'hexaméthylènediamine est de 1 à 10000 ppm avant la séparation.

6. Procédé suivant l'une des revendications 1 à 5, dans lequel la fraction pondérale de 2-aminométhylcyclopentylamine par rapport à l'hexaméthylènediamine est de 0 à 100 ppm après la séparation.
